# EUROPÄISCHE PATENTSCHRIFT

(11) **EP 2 542 205 B1**
(45) Veröffentlichungstag und Bekanntmachung des Hinweises auf die Patenterteilung: **10.04.2019**
(21) Anmeldenummer: 11712152.5
(22) Anmeldetag: 03.03.2011
(51) Int. Cl.: A61K 6/00

(54) **HAFTMITTEL FÜR ZAHNPROTHESEN UND VERFAHREN ZU SEINER HERSTELLUNG**
BONDING AGENT FOR DENTAL PROSTHESES AND METHOD FOR PRODUCING SAME
ADHÉSIF POUR PROTHÈSES DENTAIRES, ET PROCÉDÉ POUR SA PRÉPARATION

(30) Priorität: 04.03.2010 AT 3512010
(43) Veröffentlichungstag der Anmeldung: 09.01.2013
(73) Patentinhaber: Fittydent International GmbH, 7423 Pinkafeld (AT)
(72) Erfinder: WIESBAUER, Hans-Gottfried, A-4943 Geinberg (AT)
(74) Vertreter: Patentanwälte Barger, Piso & Partner
(86) Internationale Anmeldenummer: PCT/AT2011/000108
(87) Internationale Veröffentlichungsnummer: WO 2011/106817

(56) Entgegenhaltungen:
- EP-A1- 0 407 681
- EP-A2- 0 229 010
- DE-C1- 3 642 426
- US-A- 5 760 102
- US-A1- 2007 122 362
- DATABASE WPI Week 200734 Thomson Scientific, London, GB; AN 2007-361625 XP002672504, & WO 2007/039937 A1 (KOBAYASHI PHARM CO LTD) 12. April 2007 (2007-04-12)

## Beschreibung

Die Erfindung betrifft ein Haftmittel für Zahnprothesen im Ober- und Unterkieferbereich in Form einer pastösen, leicht verstreichbaren, Polyvinylacetate, Cellulosederivate, vorzugsweise mikrokristallines CMC-Granulat, Hydrokolloide, Weichmacher und ein alkoholisches Lösungsmittel enthaltenden Masse, entsprechend dem Oberbegriff des Anspruches 1 und der EP 0 407 681 A1.

EP 0 407 681 A1 offenbart einen Haftstoff für eine Hafteinlage für Zahnprothesen, der eine pastöse, leicht verformbare Masse umfasst, die Polyvinylacetat, Hydrokolloide, Carboxyme4thylcellulose enthält, wobei als Lösungsmittel Glycerintriacetat verwendet wird.

EP 0 229 010 A2 beschreibt ein Haftmittel für Zahnprothesen in Form einer pastösen , leicht verformbaren Masse, die Polyvinylacetat, einen Methylcellulose-Emulgator, ein Alginat, Carboxymethylcellulose, einen Neutralölweichmacher sowie 85%igen Ethylalkohol als Lösungsmittel umfasst.

US 5,760,102 offenbart ein Haftmittel für Zahnprothesen, das Cellulosederivate und Aloe Vera-Pulver enthält.

US 2007/122362 A1 offenbart ein Haftmittel für Zahnprothesen, das Cellulosederivate und Xanthan und wahlweise Aloe Vera Extrakt, Kamille, Stärke, Carboxymethylcellulose umfasst.

Das Dokument betreffend Database WPI, AN 2007-361625 & WO2007/039937 & JP2007/097718 offenbart ein Haftmittel für Zahnprothesen, das Carboxymethylcellulose, Ethylalkohol und einen Weichmacher, in dem Bienenwachs verwendet wird, und als Enzyme u.a. Protheasen umfasst.

DE 36 42 426 C1 beschreibt die Verwendung von Myrrhe-Tinktur in einem Imprägniermittel für Zahnprothesen.

In der AT 295 043 ist ein Cellulosederivate in einem Gemisch mit Alginsäure und/oder deren Derivaten enthaltendes Haftmittel für Zahnprothesen bekannt.

Überdies ist unter anderem in der EP 229 010 B1, der DE 35 46 367 A1 sowie der US 5,209,777 A oder der US 4,804,412 A ein Haftmittel für Zahnprothesen aus einer streichfähigen Masse geoffenbart, dass aus einer Mischung eines Alginats und eines Polyvinylacetates mit Carboxymethylcellulose, einem Methylcellulose-Emulgator und einem Neutralölweichmacher sowie einem alkoholischen Lösungsmittel, wie 85%iger Ethylalkohol besteht.

Nachteilig erwies sich bei letztgenanntem bekanntem Haftmittel das wasserunlösliche PVAC-Homopolymer bzw. das mit Säure OH Gruppen polymerisierte PVAC. Das PVAC-Homopolymer ist nur mit intensiver Reinigung von der Zahnprothese zu entfernen und das Copolymer kann Zink enthalten sowie zu Verklebungen am Gaumen führen.

Der Erfindung liegt demgemäß die Aufgabe zugrunde, die verschiedenen Mängel bekannter Haftmittel zu vermeiden, die Haftfähigkeit und die Viskosität des Haftmittels über einen langen Zeitraum zu erhalten, weiters eine Entmischung zu verhindern, Irritationen der Mundschleimhaut hintanzuhalten, Zink als Inhaltsstoff zu vermeiden und auf das Problem viel oder wenig Speichel einzugehen und die Reinigung zu erleichtern.

Die Lösung der erfindungsgemäßen Aufgabe liegt in der Schaffung eines Haftmittels der oben angegebenen Art, wobei die Masse erfindungsgemäß und wie im kennzeichnenden Teil des Anspruches 1 angegeben, als Weichmacher Raps- und/oder Sojawachs emulgiert in pflanzlichen Ölen und Fetten enthält und die Masse mit Enzymen, Myrrheharz, ätherischen Ölen, Pflanzenpulver, vorzugsweise Aloe Vera-Pulver, sowie Glyzerinacetat versetzt ist und Stärke und Xanthan, zugemischt erhält.

Eine Besonderheit vorliegender Erfindung liegt darin, dass wasserunlösliches PVAC durch eine spezielle Behandlung und im vorliegenden Fall mittels Beigaben von Enzymen und ätherischen Ölen wasseraufnahmefähig wird, zumindest soweit, dass Speichel langsam in die Klebermischung eindringen kann, von Cellulosederivaten, vorzugsweise mikrokristallinem Carboxymethylcellulose-Granulat und/oder Hydrokolloiden und Stärken aufgesaugt wird und auch die letztgenannten Pulverkomponenten zum Kleben bringt. Dies ergibt nach dem PVAC die zweite Klebestufe, sodass der Klebeeffekt bedeutend verlängert und gestärkt wird. Außerdem ergeben die Zusatzstoffe den Nebeneffekt, dass die Mundschleimhaut vor Irritationen weitgehend geschützt wird und somit auch die Haftdauer verlängert wird. Diese Mischung ist weitgehend unabhängig vom Speichelfluss, für mittleren Speichelfluss berechnet, kann aber durch Änderung der Zusammenstellung der Hydrokolloide auf viel Speichel eingestellt werden.

Nach einem speziellen Merkmal der Erfindung ist das Haftmittel dadurch gekennzeichnet, dass es eine Masse umfasst, die 35-39 Gew.-Teile nieder-/mittelmolekulare Polyvinylacetate, 17-21 Gew.-Teile 92%iges Ethanol, 0,3-0,6 Gew.-Teile Enzyme, 1.0-2.0 Gew.-Teile Glycerintriacetat, 0,3-0,6 Gew.-Teile Myrrheharzpulver, 0,2-0,4 Gew.-Teile ätherisches Kamillenöl, 2-5 Gew.-Teile Weichmacher (bestehend aus 1-1,5 Gew.-Teilen Raps- und/oder Sojawachs, 1,2-1,6 Gew.-Teilen Raps- und/oder Olivenöl, 0,2-0,6 Gew.-Teilen Kokosfett und/oder Kakaobutter), gesamt 34-40 Gew.-Teile mikrokristallines Carboxymethylcellulose-Granulat und/oder Hydrokolloide, 0,3-0,7 Gew.-Teile Aloe Vera-Pulver, 1-3 Gew.-Teile Stärken, 0,5-1,0 Gew.-Teile Xanthan enthält. Insbesondere weist die Masse erfindungsgemäß auf:
18 Gew. Teile 92%iges Ethanol
16 Gew.-Teile PVAC Homopolymer, Molekulargewicht 10.000-15.000
21 Gew.-Teile PVAC Homopolymer, Molekulargewicht 45.000-55.000
0,5 Gew.-Teile Enzyme
0,4 Gew.-Teile Myrrhe Harzpulver fein
0,3 Gew.-Teile ätherisches Kamillenöl
0,5 Gew.-Teile Aloe Vera Pulver fein
1,5 Gew.-Teile Glycerintriacetat
3,1 Gew.-Teile Weichmacher (1,3 Gew.-Teile Sojawachs, 1,4 Gew.-Teile Raps- und/oder Olivenöl, 0,4 Gew.-Teile Kokosfett und/oder Kakaobutter)
36 Gew.-Teile mikrokristallines CMC- Granulat
und/oder Hydrokolloide
2 Gew.-Teile Stärke
0,7 Gew.-Teile Xanthan

Glycerintriacetat wird bisweilen auch als Triacetin, 1,2,3-Triacetoxypropan, siehe Römpp Chemie Lexikon, 9. Auflage, 1989, bezeichnet.

Die Erfindung betrifft auch ein Verfahren zur Herstellung des Haftmittels für Zahnprothesen im Ober- und Unterkieferbereich und ist dadurch gekennzeichnet, dass getrennt voneinander eine erste Mischung aus grob vermahlenem PVAC, vorzugsweise 92%igem Ethanol, Enzymen, Harzpulvern, Glycerintriacetat, Weichmacher und ätherischem Öl und eine zweite Mischung aus Cellulosederivaten und/oder Hydrokolloiden und/oder mikrokristallinem CMC-Granulat, Xanthan, Aloe Vera Pulver und Stärke Cellulosederivate, vorzugsweise mikrokristallines CMC-Granulat, Hydrokolloide, und Xanthan und Aloe Vera-Pulver und Stärke zubereitet werden, dass die zweite Mischung der ersten Mischung zugesetzt wird, wobei unter einem eine innige Vermengung der gesamten Mischungsbestandteile vorgenommen wird, und dass schließlich unter Vakuum eine Verarbeitung des Mischgutes zu einer pastösen Masse erfolgt.

Durch die Beimengung von Enzymen, Myrrhepulver, Glycerintriacetat und ätherischen Ölen haftet das PVAC sofort auf der feuchten Mundschleimhaut, auch von Vielspeichlern, und die beigemengten Cellulosederivate, insbesondere mikrokristallines CMC-Granulat und/oder Hydrokolloide, Stärken und Xanthan und Aloe Vera Pulver steuern durch die ermöglichte Speichelaufnahme die Haftfestigkeit und Haftdauer. Die durch die Enzyme und Harz und ätherischen Öle und Glycerintriacetat ermöglichte oberflächige Wasseraufhahme von Cellulosederivaten, vorzugsweise mikrokristallinem CMC-Granulat und/oder Hydrokolloiden setzt sich innerhalb der Einbettung in PVAC verzögernd fort, ergibt somit lange Haftkraft und Haftdauer und verhindert die Ausschwemmung des Haftmittels. Das PVAC bleibt durch dieses Verfahren weich, schmiegt sich an das Zahnfleisch an und klebt zusammen mit dem Cellulosederivaten, insbesondere mikrokristallinen CMC-Granulat, und/oder Hydrokolloiden Zahnfleisch und Prothesen aneinander. Der Weichmacher erfüllt die Bedingung, auch nach dem Abgang von Alkohol das Haftmittel geschmeidig zu halten und die Zugabe von Xanthan verhindert eine Phasentrennung der alkoholischen PVAC-Lösung und der CMC- und/oder Hydrokolloid- und/oder Cellulosederivat-Anteile und hat durch die Gelierung Anteil an der hohen Haftkraft.

Zum Erhalt eines verstreichbaren, in Tuben abfüllbaren Haftmittels ist es bevorzugt, die alkoholische Lösung von lebensmittelechtem PVAC, Myrrheharz und Glycerintriacetat mit einem Weichmacher und ätherischen Ölen als erste Mischung zu fertigen, die zweite Mischung aus Cellulosederivaten, vorzugsweise mikrokristallinem CMC-Granulat, und/oder Hydrokolloiden, Stärken, Xanthan und Aloe Vera-Pulver während des Mischvorgangs kontinuierlich zuzugeben.

Diese Maßnahmen erlauben die Abfüllung des Haftmittels in Laminattuben mit Standardkanülen, um erstens die Verteilung des Haftmittels in der Prothese zu erleichtem und zweitens durch den dichten Verschluss ein Austrocknen des Haftmittels über einen längeren Zeitraum hin zu vermeiden. Durch die Verwendung neuartiger, optimale Dichtheit garantierender Tubenverschlüsse, sowie durch den Zusatz von Stärken und Xanthan wird eine Entmischung/Synärese des Haftmittels ausgeschlossen. Der Abgang von Alkohol wird ebenfalls verhindert, sodass die Wirkung des Haftmittels sowie dessen Konsistenz in der Tube über einen langen Zeitraum gewährleistet ist.

Bei der Herstellung des erfindungsgemäßen Haftmittels wird zuerst eine Mischung aus grob gemahlenem PVAC, Enzymen, Myrrheharzpulver, Glycerintriacetat und 92%igem Ethanol hergestellt. Nach dem Erkalten werden der viskosen Lösung der Weichmacher und ätherische Öle in einem Mischkneter zugesetzt und vermengt. Durch die erkaltete PVAC Lösung ergibt sich beim Mischen eine Scherwirkung, die das Einbringen des Weichmachers in die PVAC Lösung ermöglicht, wobei eine Erwärmung auf 35°C erfolgt. Als zweiter Schritt erfolgt eine Vormischung von einem kleinen Teil des Cellulosederivat-, vorzugsweise mikrokristallinen CMC-Granulat- und Hydrokolloidanteils mit der Stärke, dem Aloe Vera-Pulver und dem Xanthan. Diese Vormischung wird dem verbleibenden Anteil von Cellulosederivaten, vorzugsweise mikrokristallinem CMC-Granulat und Hydrokolloiden leicht zugemischt und diese zweite Mischung der ersten Mischung im Mischkneter während des Mischvorgangs zugegeben.

Nach erfolgter Eingabe aller Mischungkomponenten wird die Mischung durch eine externe Pumpe während des Rührens umgepumpt. Nach dem Umpumpen wird im Mischkneter Vakuum angelegt und das Mischgut gemischt, bis ca. 40-42°C erreicht sind. Diese Vorgangsweise stellt ein relativ einfaches und kostengünstiges Mischverfahren dar, wogegen andere Verfahren mit Anteigung der Pulverkomponente durch Alkohol sich wegen der auftretenden Klumpenbildung nicht bewährt haben.

Nach einer vorteilhaften Ausführungsform des erfindungsgemäßen Verfahrens werden zur Zubereitung der ersten Mischung 15-17 Gew.-Teile niedermolekulares PVAC, 20-22 Gew.-Teile mittelmolekulares PVAC, 17-21 Gew.-Teile Ethanol, 0,3-0,8 Gew.-Teile Enzyme, 0,3-0,6 Gew.-Teile feinpulveriges Myrrheharz und 1-2 Gew.-Teile Glycerintriacetat vermischt und nach Erkalten derselben 2-5 Gew.-Teile Weichmacher und 0,2-0,5 Gew.-Teile ätherische Öle, insbesondere Kamillenöl, zugemengt. Nach einem weiteren Merkmal des erfindungsgemäßen Verfahrens wird zur Herstellung der Gesamtmischung der ersten Mischung die zweite Mischung aus einer Pulvermenge von 34-40 Gew.-Teilen Cellulosederivate, vorzugsweise mikrokristallines CMC-Granulat, und/oder Hydrokolloide, 1-3 Gew.-Teilen Stärke, 0,5-1 Gew.-Teile Xanthan und 0,3-0,6 Gew.-Teile Aloe Vera-Pulver zugesetzt, worauf das gesamte Mischgut durch Energieeintragung auf 40-45°C erwärmt wird.

Der Gegenstand der Erfindung wird anhand nachfolgender Beispiele näher erläutert:

### Beispiel 1:

Es werden zur Zubereitung der ersten Mischung 15-17 Gew.-Teile niedermolekulares PVAC, 20-22 Gew.-Teile mittelmolekulares PVAC mit einer Mischung von 0,3-0,8 Gew.-Teilen Enzyme, 0,3-0,6 Gew.-Teilen Myrrheharzpulver, 1-2 Gew.-Teilen Glycerintriacetat und 2-4 Gew.-Teilen 92%igem Ethanol besprüht, kurz gemischt, auf 40°C erwärmt und 2 Stunden einwirken lassen. Hernach wird diese Masse mit 15-17 Gew.-Teilen 92%igem Ethanol unter Mischen 20 Minuten besprüht und bei 40°C acht Stunden in geschlossenem System gemischt. Nach Abkühlen auf ca. 25-35°C werden dieser Masse 2-5 Gew.-Teile Weichmacher und 0,2-0,5-Gew.-Teile ätherische Öle, insbesondere Kamillenöl, zugeführt und intensiv verrührt. Weiters wird eine Vormischung aus 1-3 Gew.-Teilen Stärke, 0,5-1 Gew.-Teilen Xanthan und 1-4 Gew.-Teilen mikrokristallinem CMC-Granulat, Hydrokolloiden und 0,3-0,6 Gew.-Teilen Aloe Vera-Pulver hergestellt, diese Vormischung den 33-36 Gew.-Teilen mikrokristallinem CMC-Granulat und Hydrokolloiden kurz zugemischt - zweite Mischung - und diese Menge kontinuierlich der ersten Mischung unter intensivem Rühren zugegeben. Es handelt sich hier um ein spezielles Mischverfahren mit für den Mischer genau festgelegten Drehzahlen und vorbestimmter Mischdauer, wobei die Masse während des Rührens mit einer Monopumpe durch einen externen Rohrmischer umgepumpt und Vakuum angelegt wird. Dadurch wird höchste Homogenität und Verstreichfähigkeit für die Haftmittelmasse erreicht.

### Beispiel 2:

Es wird eine erste Mischung aus 16 Gew.-Teilen PVAC Homopolymer 10.000-15.000 mol, 21 Gew.-Teilen PVAC Homopolymer 45.000-55.000 mol, 18 Gew.-Teilen 92%igem Ethanol, 0,5 Gew.-Teilen Enzyme, 0,4 Gew.-Teilen Myrrheharzpulver, 1,5 Gew.-Teilen Glycerintriacetat bei 60°C Mischtemperatur und ca. 10 Stunden Mischzeit hergestellt. Nach dem Erkalten wird eine Laborprobe entnommen, diese muss einen Feststoffgehalt von 66-68% aufweisen. Die so hergestellte erste Mischung wird nach dem Erkalten in einem Mischkneter mit 3,1 Gew.-Teilen Weichmachern und 0,3 Gew.-Teilen ätherischem Kamillenöl verrührt, bis die Mischung milchig weiß erscheint und ca. 35°C erreicht hat. Zwischendurch wird die zweite Mischung vorbereitet:
Eine Vormischung mit 2 Gew.-Teilen mikrokristallinem CMC-Granulat und/oder Hydrokolloiden, 2 Gew.-Teilen Stärke, 0,7 Gew.-Teilen Xanthan und 0,5 Gew.-Teilen Aloe Vera-Pulver wird zubereitet und mit 34 Gew.-Teilen mikrokristallinem CMC-Granulat und/oder Hydrokolloiden kurz vermengt und dieses Gemenge wird kontinuierlich unter intensivem Rühren der ersten Mischung zugegeben. Nach ca. 20 Minuten Mischzeit mit Umpumpen der Masse wird Vakuum angelegt und weitere 10 Minuten gemischt. Hernach hat das Mischgut eine optimale Konsistenz und Temperatur von ca. 40-42°C für die Abfüllung erreicht. Der Verlust von Ethanol ist durch eine geschlossene Verfahrensführung und die Bindung desselben an das PVAC vernachlässigbar.

## Patentansprüche

1. Haftmittel für Zahnprothesen im Ober- und Unterkieferbereich in Form einer pastösen, leicht verstreichbaren, Polyvinylacetate, Cellulosederivate, vorzugsweise mikrokristallines CMC-Granulat, Hydrokolloide, Weichmacher und ein alkoholisches Lösungsmittel enthaltenden Masse, **dadurch gekennzeichnet, dass** die Masse als Weichmacher Raps- und/oder Sojawachs emulgiert in pflanzlichen Ölen und Fetten enthält und die Masse mit Enzymen, Myrrheharz, ätherischen Ölen, Pflanzenpulver, vorzugsweise Aloe Vera-Pulver, sowie Glycerintriacetat versetzt ist und Stärke und Xanthan zugemischt enthält,

2. Haftmittel nach Anspruch 1, **dadurch gekennzeichnet, dass** es eine Masse umfasst, die 35-39 Gew.-Teile nieder- und mittelmolekulares PVAC, 17-21 Gew.-Teile Ethanol, 0,3-0,8 Gew.-Teile Enzyme, 0,3-0,6 Gew.-Teile Myrrheharzpulver, 1-2 Gew.-Teile Glycerintriacetat, 2-5 Gew.-Teile Weichmacher, 0,2-0,4 Gew.-Teile ätherische Öle/Kamillenöl, 34-40 Gew.-Teile mikrokristallines CMC-Granulat und/oder Hydrokolloide, 0,3-0,7 Gew.-Teile Aloe Vera-Pulver, 1-3 Gew.-Teile Stärke, 0,5-1 Gew.-Teile Xanthan aufweist.

3. Haftmittel nach Anspruch 2, **dadurch gekennzeichnet, dass** die Masse 16 Gew.-Teile Polyvinylacetat-Homopolymere mit einem Molekulargewicht zwischen 10,000-15.000, 21 Gew.-Teile Polyvinylacetat- Homopolymere mit einem Molekulargewicht zwischen 45.000-55.000, 18 Gew.-Teile 92%iges Ethanol, 0,5 Gew.-Teile Enzyme, 0,4 Gew.-Teile Myrrheharz-Feinpulver, 1,5 Gew.-Teile Glycerintriacetat, 3,1 Gew.-Teile Weichmacher, 0,3 Gew.-Teile ätherisches Kamillenöl, 36 Gew.-Teile mikrokristallines CMC-Granulat, und/oder Hydrokolloide, 2 Gew.-Teile Stärke, 0,7 Gew.-Teile Xanthan und 0,5 Gew.-Teile Aloe Vera-Pulver.

4. Haftmittel nach Anspruch 2, **dadurch gekennzeichnet, dass** der Weichmacher aus 1- 1,5 Gew-.Teilen Raps- und/oder Sojawachs, 1,2-1,6 Gew.-Teilen Raps- und/oder Olivenöl und 0,2-0,6 Gew.-Teilen Kokosfett und/oder Kakaobutter besteht.

5. Haftmittel nach Anspruch 3, **dadurch gekennzeichnet, dass** der Weichmacher aus 1,3-Gew.-Teilen Sojawachs, 1,4 Gew.-Teilen Raps- und/oder Olivenöl und 0,4 Gew.-Teilen Kokosfett und/oder Kakaobutter besteht.

6. Haftmittel nach einem der Ansprüche 1-3,**dadurch gekennzeichnet, dass** die Enzyme Hydrolasen sind.

7. Haftmittel nach Anspruch 3, **dadurch gekennzeichnet, dass** die Hydrolasen Lipasen und/oder Proteasen sind.

8. Haftmittel nach Anspruch 1 oder 2, **dadurch gekennzeichnet, dass** das alkoholische Lösungsmittel 92%iges Ethanol ist.

9. Verfahren zur Herstellung eines Haftmittels für Zahnprothesen im Ober- und Unterkieferbereich nach einem der Ansprüche 1 bis 8, **dadurch gekennzeichnet, dass** getrennt voneinander eine erste Mischung aus grob vermahlenem PVAC, vorzugsweise 92%igem Ethanol, Enzymen, Harzpulvern, Glycerintriacetat, Weichmacher und ätherischem Öl und eine zweite Mischung aus Cellulosederivaten, vorzugsweise mikrokristallinem CMC-Granulat und/oder Hydrokolloiden, Xanthan, Aloe Vera-Pulver und Stärke zubereitet werden, dass die erste und zweite Mischung miteinander vereinigt wird, wobei unter einem eine innige Vermengung der gesamten Mischungsbestandteile vorgenommen wird, und dass schließlich unter Vakuum eine Verarbeitung des Mischgutes zu einer pastösen Masse erfolgt.

10. Verfahren nach Anspruch 9, **dadurch gekennzeichnet, dass** zur Zubereitung der ersten Mischung 15-17 Gew.-Teile niedermolekulares PVAC, 20-22 Gew.-Teile mittelmolekulares PVAC, 17-21 Gew.-Teile 92%iges Ethanol, 0,3-0,8 Gew. Teile Enzyme, 0,3-0,6 Gew.-Teile Myrrheharzpulver, 1-2 Gew.-Teile Glycerintriacetat vermischt werden und nach Lösung des PVAC 2-5 Gew.-Teile Weichmacher und 0,2-0,4 Gew.-Teile ätherisches Kamillenöl zugemengt werden.

11. Verfahren nach Anspruch 9, **dadurch gekennzeichnet, dass** die zweite Mischung aus einer Pulvermenge von 34-40 Gew.-Teilen Cellulosederivaten, vorzugsweise mikrokristallinem CMC-Granulat und/oder Hydrokolloiden, 1-3 Gew.-Teilen Stärke, 0,5-1 Gew.- Teilen Xanthan, 0,3-0,7 Gew.-Teile Aloe Vera Pulver zubereitet und der ersten Mischung zugesetzt wird, worauf das gesamte Mischgut durch Energieeintragung auf ca. 40-45°C erwärmt wird.

12. Verfahren nach Anspruch 10, **dadurch gekennzeichnet, dass** zur Zubereitung der ersten Mischung 16 Gew.-Teile niedermolekulares PVAC-Homopolymer, 21 Gew.-Teile mittelmolekulares PVAC-Homopolymer, 0,5 Gew.-Teile Enzyme, 0,4 Gew.-Teile Myrrheharzpulver, 1,5 Gew.-Teile Glycerintriacetat und 18 Gew.-Teile 92%iges Ethanol vermischt und erwärmt werden und nach Lösung des PVAC 3,1 Gew.-Teile Weichmacher und 0,3 Gew.-Teile ätherisches Öl/Kamillenöl zugemengt werden.

13. Verfahren nach Anspruch 11, **dadurch gekennzeichnet, dass** der ersten Mischung eine zweite Mischung aus einer Pulvermenge von 36 Gew.-Teilen Cellulosederivaten und/oder Hydrokolloiden und/oder mikrokristallinem CMC-Granulat, 2 Gew.-Teilen Stärke, 0,7 Gew.-Teilen Xanthan und 0,5 Gew.-Teilen Aloe Vera Pulver zugeführt wird.

14. Verfahren nach einem der Ansprüche 9 bis 11, **dadurch gekennzeichnet, dass** 100 Gew.- Teile der PVAC umfassenden ersten Mischung mit 64,5 Gew.-Teilen der die zweite Mischung bildenden Pulvergemenge intensiv vermischt, umgepumpt und zur Weiterverarbeitung unter Vakuum gelegt werden.

## Claims

1. Bonding agent for dental prostheses in the upper and lower jaw region in the form of a paste-like, easily spreadable mass containing polyvinyl acetates, cellulose derivatives, preferably microcrystalline CMC granulate, hydrocolloids, softener and an alcoholic solvent, **characterised in that** the mass contains rapeseed wax and/or soya wax emulsified in plant oils and fats as the softener, and the mass is mixed with enzymes, myrrh resin, essential oils, plant powder, preferably Aloe Vera powder, and glycerine triacetate and contains starch and xanthan mixed in.

2. Bonding agent according to claim 1, **characterised in that** it comprises a mass which has 35-39 parts by weight low-molecular weight and medium-molecular weight PVAC, 17-21 parts by weight ethanol, 0.3-0.8 parts by weight enzymes, 0.3-0.6 parts by weight myrrh resin powder, 1-2 parts by weight glycerine triacetate, 2-5 parts by weight softener, 0.2-0.4 parts by weight essential oils/chamomile oil, 34-40 parts by weight microcrystalline CMC granulate and/or hydrocolloids, 0.3-0.7 parts by weight Aloe Vera powder, 1-3 parts by weight starch, 0.5-1 parts by weight xanthan.

3. Bonding agent according to claim 2, **characterised in that** the mass 16 parts by weight polyvinyl acetate homopolymers having a molecular weight between 10,000 and 15,000, 21 parts by weight polyvinyl acetate homopolymers having a molecular weight between 45,000 and 55,000, 18 parts by weight 92% ethanol, 0.5 parts by weight enzymes, 0.4 parts by weight myrrh resin fine powder, 1.5 parts by weight glycerine triacetate, 3.1 parts by weight softener, 0.3 parts by weight essential chamomile oil, 36 parts by weight microcrystalline CMC granulate, and/or hydrocolloids, 2 parts by weight starch, 0.7 parts by weight xanthan and 0.5 parts by weight Aloe Vera powder.

4. Bonding agent according to claim 2, **characterised in that** the softener consists of 1-1.5 parts by weight rapeseed wax and/or soya wax, 1.2-1.6 parts by weight rapeseed oil and/or olive oil and 0.2-0.6 parts by weight coconut oil and/or cocoa butter.

5. Bonding agent according to claim 3, **characterised in that** the softener consists of 1.3 parts by weight soya wax, 1.4 parts by weight rapeseed oil and/or olive oil and 0.4 parts by weight coconut oil and/or cocoa butter.

6. Bonding agent according to one of claims 1-3, **characterised in that** the enzymes are hydrolases.

7. Bonding agent according to claim 3, **characterised in that** the hydrolases are lipases and/or proteases.

8. Bonding agent according to claim 1 or 2, **characterised in that** the alcoholic solvent is 92% ethanol.

9. Method for producing a bonding agent for dental prostheses in the upper and lower jaw region according to one of claims 1 to 8, **characterised in that**, separate from each other, a first mixture made of roughly milled PVAC, preferably 92% ethanol, enzymes, resin powders, glycerine triacetate, softener and essential oil and a second mixture made of cellulose derivatives, preferably microcrystalline CMC granulate and/or hydrocolloids, xanthan, Aloe Vera powder and starch are prepared, the first and second mixtures are combined with each other, wherein an inner blending of all the mixture components simultaneously takes place, and finally, a processing of the mix takes place in vacuo to form a paste-like mass.

10. Method according to claim 9, **characterised in that**, in order to prepare the first mixture, 15-17 parts by weight low-molecular weight PVAC, 20-22 parts by weight medium-molecular weight PVAC, 17-21 parts by weight 92% ethanol, 0.3-0.8 parts by weight enzymes, 0.3-0.6 parts by weight myrrh resin powder, 1-2 parts by weight glycerine triacetate are mixed in and, after dissolving the PVAC, 2-5 parts by weight softener and 0.2-0.4 parts by weight essential chamomile oil are added in.

11. Method according to claim 9, **characterised in that** the second mixture made of a quantity of powder of 34-40 parts by weight cellulose derivatives, preferably microcrystalline CMC granulate and/or hydrocolloids, 1-3 parts by weight starch, 0.5-1 parts by weight xanthan, 0.3-0.7 parts by weight Aloe Vera powder is prepared and added to the first mixture, whereupon the total mix is heated to c. 40-45°C by means of energy input.

12. Method according to claim 10, **characterised in that**, in order to prepare the first mixture, 16 parts by weight low-molecular weight PVAC homopolymers, 21 parts by weight medium-molecular weight PVAC homopolymer, 0.5 parts by weight enzymes, 0.4 parts by weight myrrh resin powder, 1.5 parts by weight glycerine triacetate and 18 parts by weight 92% ethanol are mixed in and heated and, after dissolving the PVAC, 3.1 parts by weight softener and 0.3 parts by weight essential oil/chamomile oil are added in.

13. Method according to claim 11, **characterised in that** a second mixture made of a quantity of powder of 36 parts by weight cellulose derivatives and/or hydrocolloids and/or microcrystalline CMC granulate, 2 parts by weight starch, 0.7 parts by weight xanthan and 0.5 parts by weight Aloe Vera powder is supplied to the first mixture.

14. Method according to one of claims 9 to 11, **characterised in that** 100 parts by weight of the first mixture comprising PVAC, having 64.5 parts by weight of the quantity of powder forming the second mixture, are intensively mixed, pumped and placed in vacuo for further processing.

## Revendications

1. Adhésif pour prothèses dentaires dans la région de la mâchoire supérieure et inférieure sous la forme d'une composition pâteuse, facilement étalable contenant des acétates de polyvinyle, des dérivés de cellulose, de préférence des granulés de CMC microcristallins, des hydrocolloïdes, des plastifiants et un solvant alcoolique, **caractérisé en ce que** la composition contient en tant que plastifiant de la cire de colza et/ou de soja émulsionnée dans des huiles et des graisses végétales et la composition est mélangée à des enzymes, de la résine de myrrhe, des huiles essentielles, une poudre végétale, de préférence de la poudre d'aloe vera, ainsi que du triacétate de glycérol et contient en mélange de l'amidon et de la gomme xanthane.

2. Adhésif selon la revendication 1, **caractérisé en ce qu'**il comprend une composition qui présente de 35 à 39 parties en poids de PVAC de poids moléculaire bas et moyen, de 17 à 21 parties en poids d'éthanol, de 0,3 à 0,8 partie en poids d'enzymes, de 0,3 à 0,6 partie en poids de poudre de résine de myrrhe, de 1 à 2 parties en poids de triacétate de glycérol, de 2 à 5 parties en poids de plastifiant, de 0,2 à 0,4 partie en poids d'huiles essentielles/huile essentielle de camomille, de 34 à 40 parties en poids de granulés de CMC microcristallins et/ou d'hydrocolloïdes, de 0,3 à 0,7 partie en poids de poudre d'aloe vera, de 1 à 3 parties en poids d'amidon, de 0,5 à 1 partie en poids de gomme xanthane.

3. Adhésif selon la revendication 2, **caractérisé en ce que** la composition présente 16 parties en poids d'homopolymères d'acétate de polyvinyle ayant un poids moléculaire compris entre 10 000 et 15 000, 21 parties en poids d'homopolymères d'acétate de polyvinyle ayant un poids moléculaire compris entre 45 000 et 55 000, 18 parties en poids d'éthanol à 92 %, 0,5 partie en poids d'enzymes, 0,4 partie en poids de poudre fine de résine de myrrhe, 1,5 parties en poids de triacétate de glycérol, 3,1 parties en poids de plastifiant, 0,3 partie en poids d'huile essentielle de camomille, 36 parties en poids de granulés de CMC microcristallins et/ou d'hydrocolloïdes, 2 parties en poids d'amidon, 0,7 partie en poids de gomme xanthane et 0,5 partie en poids de poudre d'aloe vera.

4. Adhésif selon la revendication 2, **caractérisé en ce que** le plastifiant est constitué de 1 à 1,5 parties en poids de cire de colza et/ou de soja, de 1,2 à 1,6 parties en poids d'huile de colza et/ou d'olive et de 0,2 à 0,6 partie en poids de graisse de coco et/ou de beurre de cacao.

5. Adhésif selon la revendication 3, **caractérisé en ce que** le plastifiant est constitué de 1,3 parties en poids de cire de soja, de 1,4 parties en poids d'huile de colza et/ou d'olive et de 0,4 partie en poids de graisse de coco et/ou de beurre de cacao.

6. Adhésif selon l'une des revendications 1 à 3, **caractérisé en ce que** les enzymes sont des hydrolases.

7. Adhésif selon la revendication 3, **caractérisé en ce que** les hydrolases sont des lipases et/ou des protéases.

8. Adhésif selon la revendication 1 ou 2, **caractérisé en ce que** le solvant alcoolique est de l'éthanol à 92 %.

9. Procédé de fabrication d'un adhésif pour prothèses dentaires dans la région de la mâchoire supérieure et inférieure selon l'une des revendications 1 à 8, **caractérisé en ce qu'**un premier mélange de PVAC grossièrement broyé, de préférence d'éthanol à 92 %, d'enzymes, de poudres de résine, de triacétate de glycérol, de plastifiant et d'huile essentielle et un second mélange de dérivés de cellulose, de préférence de granulés de CMC microcristallins et/ou d'hydrocolloïdes, de gomme xanthane, de poudre d'aloe vera et d'amidon sont préparés séparément, que les premier et second mélanges sont combinés entre eux, un mélange intime de l'ensemble des constituants du mélange en un seul étant effectué, et que, finalement, le mélange est traité sous vide pour obtenir une composition pâteuse.

10. Procédé selon la revendication 9, **caractérisé en ce que** de 15 à 17 parties en poids de PVAC de bas poids moléculaire, de 20 à 22 parties en poids de PVAC de poids moléculaire moyen, de 17 à 21 parties en poids d'éthanol à 92 %, de 0,3 à 0,8 partie en poids d'enzymes, de 0,3 à 0,6 partie en poids de poudre de résine de myrrhe, de 1 à 2 parties en poids de triacétate de glycérol sont mélangées pour préparer le premier mélange et de 2 à 5 parties en poids de plastifiant et de 0,2 à 0,4 partie en poids d'huile essentielle de camomille sont ajoutées après dissolution du PVAC.

11. Procédé selon la revendication 9, **caractérisé en ce que** le second mélange est préparé à partir d'une quantité de poudre de 34 à 40 parties en poids de dérivés de cellulose, de préférence de granulés de CMC microcristallins et/ou d'hydrocolloïdes, de 1 à 3 parties en poids d'amidon, de 0,5 à 1 partie en poids de gomme xanthane, de 0,3 à 0,7 partie en poids de poudre d'aloe vera et ajouté au premier mélange, le mélange entier étant ensuite chauffé à environ 40-45 °C par apport d'énergie.

12. Procédé selon la revendication 10, **caractérisé en ce que** 16 parties en poids d'homopolymère de PVAC de bas poids moléculaire, 21 parties en poids d'homopolymère de PVAC de poids moléculaire moyen, 0,5 partie en poids d'enzymes, 0,4 partie en poids de poudre de résine de myrrhe, 1,5 parties en poids de triacétate de glycérol et 18 parties en poids d'éthanol à 92 % sont mélangées et chauffées pour préparer le premier mélange et 3,1 parties en poids de plastifiant et 0,3 partie en poids d'huile essentielle/huile essentielle de camomille sont ajoutées après dissolution du PVAC.

13. Procédé selon la revendication 11, **caractérisé en ce qu'**on ajoute au premier mélange un second mélange d'une quantité de poudre de 36 parties en poids de dérivés de cellulose et/ou d'hydrocolloïdes et/ou de granulés de CMC microcristallins, de 2 parties en poids d'amidon, de 0,7 partie en poids de gomme xanthane et de 0,5 partie en poids de poudre d'aloe vera.

14. Procédé selon l'une des revendications 9 à 11, **caractérisé en ce que** 100 parties en poids du premier mélange comprenant du PVAC sont mélangées de manière intensive avec 64,5 parties en poids du mélange de poudres formant le second mélange, mises en circulation par pompage et placées sous vide pour traitement ultérieur.
